# EUROPEAN PATENT SPECIFICATION

(11) **EP 4 378 431 B1**
(45) Date of publication and mention of the grant of the patent: **22.07.2026**
(21) Application number: 24166233.7
(22) Date of filing: 20.11.2020
(51) Int. Cl.: A61F 5/44

(54) **PORTABLE URINE COLLECTION SYSTEM AND RELATED METHODS**
TRAGBARES URINSAMMELSYSTEM UND ZUGEHÖRIGE VERFAHREN
SYSTÈME DE COLLECTE D'URINE PORTABLE ET PROCÉDÉS ASSOCIÉS

(30) Priority: 21.11.2019 US 201962938447 P
(43) Date of publication of application: 05.06.2024
(62) Divisional of application: 20817592.7
(73) Proprietor: Purewick Corporation, El Cajon, California 92020 (US)
(72) Inventor: HUGHET, James David, Conyers, Georgia (US); DAW, Kyle, Smyrna, Georgia 30080 (US); HINESLEY, Hannah, Monticello, Georgia 31064 (US); FERNANDEZ, Rodrigo, Loganville, Georgia 30052 (US); HIETT, Ginger, Covington, Georgia 30016 (US); BOWLES, Caitlin, Atlanta, Georgia 30313 (US); CISNEROS, Juan Alejandro Saavedra, Loganville, Georgia 30052 (US); MADIGAN, Henri, Monroe, Georgia 30656 (US); SALIFU, Hassana, Roswell, Georgia 30076 (US); HUANG, Ping, Libertyville, Georgia 60048 (US); CHEN, Jingkuang, Rochester, New York 14618 (US)
(74) Representative: Hoffmann Eitle

(56) References cited:
- EP-A1- 0 610 638
- WO-A1-03/079942
- KR-B1- 101 432 639
- US-A- 4 631 061

## Description

### BACKGROUND

An individual may have limited or impaired mobility such that typical urination processes are challenging or impossible. For example, the individual may have had surgery or may have a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections.

EP-A-610638 discloses an assembly to be worn by a person who suffers from urinary incontinence, the assembly including a urine collector unit and a urine storage container, a flushing liquid container and pump and an air pump for sweeping the urine collector unit with air after it has been washed with the flushing liquid.

Thus, users and manufacturers of fluid collection devices continue to seek new and improved devices, systems, and methods to collect urine.

### SUMMARY

The invention is defined in claim 1 below. The dependent claims are directed to optional features and preferred embodiments. Disclosed herein are fluid collection devices and methods of using fluid collection devices. A portable urine collection system, according to claim 1, is disclosed.

A method of assembling such a portable urine collection system is disclosed in claim 8.

Also disclosed below is a portable urine collection system which includes a container including an interior region, a modular pump in fluid communication with the interior region, and a backpack. The modular pump is configured to pull a vacuum and draw urine from a urine collection device positioned proximate to a urethra of a user into the interior region of the chamber. The backpack is configured to be worn by the user and sized and dimensioned to hold the container and the modular pump therein when being worn by the user.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1** is a block diagram of a portable urine collection system, according to the invention.
**FIG. 2** is a block diagram of a portable urine collection system, not being part of the invention.
**FIG. 3** is a flow diagram of a method for assembling a portable urine collection system.

### DETAILED DESCRIPTION

Disclosed herein are fluid collection devices and methods of using the same. The devices and systems disclosed herein are configured to collect fluids from an individual. The fluids collected by the fluid collection devices may include at least one of urine, vaginal discharge, penile discharge, reproductive fluids, blood, sweat, or other bodily fluids.

Conventional urine collection systems are typically designed for patients confined to a bed and, accordingly, typically have a fixed architecture including a urine collection device, a conduit, a urine collection container, and a pump. For example, in a conventional urine collection system, if a pump of the urine collection system is no longer functional, additional elements of the urine collection system may also need to be replaced in addition to the pump. Moreover, conventional urine collection systems may also be limited to and/or compatible with a single type or size of pump, and a user or caregiver is prohibited from interchanging the pump with a different pump to meet the needs of the user or certain circumstances. These aspects of conventional urine collection systems can be detrimental to the environment and increase unnecessary waste.

Many embodiments presented herein include a modular architecture that allows one or more components to be replaced by a similar component or a component having features desired for specific activities or a specific user. The one or more components may be configured for replacement by an end user after an original component has been used, malfunctions, or does not operate with the end user as desired. For example, a female urine collection device may be removed and replaced with a male urine collection device, a smaller urine collection device can be replaced with a larger urine collection device, a smaller power source can be replaced with a larger power source, one pump can be replaced with another pump, a dysfunctional pump or power source can be replaced with a functioning pump or power source, a soiled or kinked conduit can be replaced with a clean conduit, a soiled urine collection device can be replaced with a clean urine collection device, and so on. Embodiments of modular urine collection systems described herein also allow a user to reuse one or more of the pump, the power source (e.g., battery), the controller, or the three-way valves, while one or more of the urine collection device, the conduit, or the collection bag may be disposable. A disposable device, such as a disposable modular pump, includes a device that may be manually disconnected from the system by a user or caregiver without damaging the remaining system, and then placed in the garbage or rubbish bin. The one or more components may be replaced with different components by the user at the convenience or preference of the user. In embodiments, connectors between modular components may be generally universal, allowing each component to be replaced with a suitable counterpart.

Conventional urine collection systems may be sized and dimensioned for a user to use the conventional urine collection system anywhere but in bed next to a wall-mounted power source but nowhere else. However, incontinent ambulatory users have need for a urine collection system that allows them to carry about more normal activities without the embarrassment of soiling his/her clothes.

The modular architecture of the urine collection systems described herein also may be portable by the user or caregiver without assistance of a motorized vehicle. A portable urine collection system is sized, dimensioned, and weighted to allow a user or caregiver to carry the entire urine collection system with, for example, only the assistance of a backpack and/or a sleeve. Thus, the portable and modular architecture of urine collection systems described herein also allows the patient or end user to use the system in a variety of settings, such as a wheelchair or ambulatory. For ambulatory users, in many of the urine collection systems described herein, the pump, the battery, and the fluid collection bag are sized and dimensioned to be simultaneously carried by the user when the urine collection device is positioned at least proximate to the urethra of the user. Also for ambulatory users, in many of the urine collection systems describe herein the pump and the power supply are sized and dimensioned to be detachably secured proximate to a waist of the user and the urine collection bag is sized and dimensioned to be positioned within a pocket of a leg sleeve worn on the leg of the user.

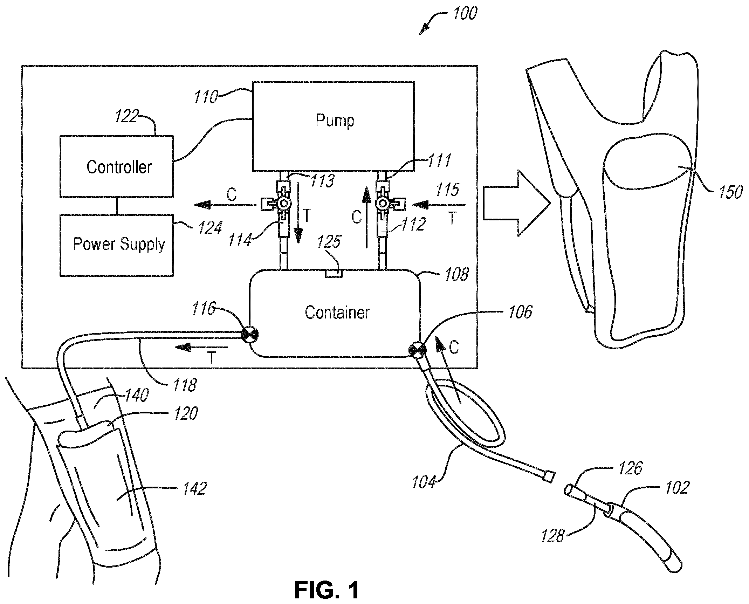

**FIG. 1** is a block diagram of a portable urine collection system 100, according to an embodiment. The urine collection system 100 includes a urine collection device 102, a first conduit 104, a container 108, a pump 110, a second conduit 118, a collection bag 120, a controller 122, and a power supply 124. While the urine collection device 102 shown in **FIG. 1** includes a female urine collection device, the urine collection device 102 may instead include a male urine collection device. PCT International Application No. PCT/US2019/029616, for example, describes various embodiments of both male and female fluid collection devices. Moreover, the urine collection device 102 may be interchangeable in the urine collection system 100 between different types, varieties, and sizes of male or female urine collection devices. The urine collection device 102 is configured to be positioned proximate or adjacent to a urethra of a user. Generally, the urine collection device 102 may include a surface sized to be positioned proximate or adjacent to the urethra and configured to wick urine or other fluids away from the user. Urine or other fluids may be wicked from the surface to a reservoir in the urine collection device 102.

The urine collection system 100 also includes a first conduit 104. The first conduit 104 is configured to provide fluid communication between the pump 110 and the urine collection device 102, and may include a flexible tube. In many embodiments, the fluid collection device 102 includes a connector 126 configured to connect to the first conduit 104. The fluid collection device 102 also may include an additional conduit 128 connecting the connector 126 and the body of the fluid collection device 102. The additional conduit 128 may provide fluid communication with the reservoir of the urine collection device 102 or an interior portion of the urine collection device 102 to allow urine to be withdrawn from the urine collection device 102 through the additional conduit 128. When connected, the additional conduit 128 provides fluid communication between the first conduit 104 and the urine collection device 102. In some embodiments, the first conduit 104 may connect directly to the urine collection device 102 to provide fluid communication with the reservoir of the urine collection device 102 or an interior portion of the urine collection device 102 to allow urine to be withdrawn from the urine collection device through the first conduit 104.

The urine collection system 100 also includes a container 108 that may be generally rigid, according to an embodiment. For example, the container 108 may be more rigid than the collection bag. A generally rigid container 108 of this disclosure is configured to hold its shape under the vacuum and positive pressures exerted by the pump 110. In some embodiments, the container 108 is sized to hold a smaller volume of fluid or air than the collection bag 120. For example, the container 108 may be sized to hold less than 500 mL of fluid or air, while the collection bag 120 may be sized to hold at least 1000 mL of fluid or air. In some embodiments, the container 108 may be sized to hold about 200 mL to about 400 mL of fluid or air and the collection bag 120 may be sized to hold at least about 1000 mL or at least about 1500 mL of fluid or air.

The first conduit 104 is connected to the container 108, according to an embodiment. The urine collection system 100 also may include a first check valve 106 providing fluid communication from the first conduit 104 into the container 108, while preventing fluid communication from the container 108 into the first conduit 104. For example, upon activation of the pump 110, a vacuum formed in the container 108 may pull a vacuum on the first conduit 104, thereby pulling fluid from at least one of the first conduit 104 or the urine collection device 102 and into the container 108. The first check valve 106, however, also may prevent fluid collected in the container 108 from passing through the first check valve 106 into the first conduit 104.

The container 108 also is connected to the second conduit 118. The urine collection system 100 may include a second check valve 116 providing fluid communication from the container 108 into the second conduit 118, while preventing fluid communication from the second conduit 118 into the container 108. For example, when the pump 110 is activated and a vacuum is formed in the container 108, the second check valve 116 may prevent fluid communication from the second conduit 118 into the container 108, thereby preventing any fluids in the second conduit 118 or the collection bag 120 from passing through the second check valve 116 and entering the container 108. When a predetermined positive pressure is formed in the container 108 upon activation of the pump, the second check valve 116 may provide fluid communication from the container 108 into the second conduit 118, thereby forcing fluid in the container 108 through the second check valve 116 into the second conduit 118 and into the collection bag 120.

The second conduit 118 is connected to the container 108 and configured to provide fluid communication between the container 108 and the collection bag 120 when the predetermined positive pressure is formed in the container 108 to force fluid in the container 108 through the second check valve 116 into the second conduit 118. The second conduit 118 may include a flexible tube. The second conduit 118 may be connected directly to the collection bag 120. In some embodiments, the second conduit 118 is connected to a connector that is connected to the collection bag 120. An additional conduit may connect the connector to the collection bag 120. The additional conduit provides fluid communication with an interior portion of the collection bag 120 to allow urine to be forced through the second conduit 118 into the collection bag 120. When connected, the additional conduit provides fluid communication between the second conduit 118 and the collection bag 120.

The collection bag 120 may be a collapsible or expandable bag sized to hold more fluid or air than the container 108. For example, the collection bag 120 may be generally flat when empty, but may expand when urine or other fluid is forced into the collection back 120. The collection bag 120 also may include one or more vents. The one or more vents may be configured to allow air in the collection bag 120 to exit the collection bag 120, but prevent fluid in the collection bag 120 from exiting the collection bag 120 through the one or more vents. The collection bag 120 also may include a discharge port for emptying fluid from the collection bag 120.

The pump 110 of the urine collection system 110 may include any of a variety of pumps, such as a diaphragm pump. The pump 110 may include multiple flow rates that allow a user or wearer to select a desired or preferred flow rate. For example, a higher flow rate may be required to force fluid out of the container 108 than is required to pull fluid into the container 108. As noted above, the pump 110 may include a removable or modular pump that allows a user to remove the pump 110 and attach a different pump to the urine collection system 110. The pump 110 also may include a portable pump that is transportable by a user or wearer. For example, the pump 110 may be sized to carry in a backpack 150 or attached to the clothing of a user or wearer.

The pump 110 also is configured to pull a vacuum in the first conduit 104 to pull fluid from the urine collection device 102. The pump 110 also may be configured force fluid through the second conduit 118 into the collection bag 120. In some embodiments, the pump 110 is a reversible pump, configured to selectively pull fluid or air in and push fluid or air out. For example, the pump 110 may be fluidly coupled to the container 108 and may be reversible between a first pump action and a second pump action. During the first pump action, the pump 110 pulls a vacuum in the container 108 to pull urine or other fluids through the first check valve 106 into the container 108. During the second pump action, the pump 110 creates a positive pressure in the container 108, thereby forcing urine through the second check valve 116 into the second conduit 118 and the collection bag 120.

In many embodiments, the pump 110 may include an air intake 111 and an air exhaust 113. Upon activation of the pump 110, the air intake 111 may draw air into the pump 110 through the air intake 111 and the air exhaust 113 may force air out of the pump 110 through the air exhaust 113. The urine collection system 100 also may include a first three-way valve 112 and a second three-way valve 114. The first three-way valve 112 may be connected to the air intake 111 and a first port on the container 108, while the second three-way valve 112 may be connected to the air exhaust 113 and a second port on the container 108.

The first three-way valve 112 and the second three-way valve 114 are each operable in a first configuration and a second configuration and may, in selected configurations, provide fluid communication between the pump 110 and the container. The first three-way valve 112 includes a first configuration that provides fluid communication between the container 108 and the pump 110. The configuration of the first three-way valve 112 also may be selectively changed or switched to a second configuration that prevents fluid communication between the container 108 and the pump 110, but provides fluid communication between the pump 110 and ambient air 115 from outside or around the first three-way valve 112. The second three-way valve 114 includes a first configuration that provides fluid communication between the container 108 and the pump 110. The configuration of the second three-way valve 114 also may be selectively changed or switched to a second configuration that prevents fluid communication between the container 108 and the pump 110, but provides fluid communication between the pump 110 and the ambient air 115.

Upon activation of the pump 110, the first three-way valve 112 and the second three-way valve 144 allow the pump 110 to either pull a vacuum on the first conduit 104 to pull fluid from the urine collection device 102 into the container 108 or force fluid in the container 108 through the second conduit 118 into the collection bag 120. The urine collection system 100, then, can alternate between a collection mode C and a transfer mode T. In the collection mode C, the first three-way valve 112 is in the first configuration (that provides fluid communication between the container 108 and the pump 110) and the second three-way valve 114 is in the second configuration (that prevents fluid communication between the container 108 and the pump 110, but provides fluid communication between the pump 110 and the ambient air 115). Activation of the pump 110 when the urine collection system 100 is in the collection mode C pulls air through the first three-way valve 112 and the air intake 111 into the pump 110 from the container 108 and exhausts the air through the air exhaust 113 and the second three-way valve 114 to the ambient air 115 outside or around the second three-way valve 114. This flow of air pulls a vacuum on the urine collection device 102 and the first conduit 104 sufficient to pull fluid from the urine collection device 102 and the first conduit 104 through the first check valve 106 into the container 108.

In the transfer mode T, the first three-way valve 112 is in the second configuration (that prevents fluid communication between the container 108 and the pump 110, but provides fluid communication between the pump 110 and ambient air 115 from outside or around the first three-way valve 112) and the second three-way valve 114 is in the first configuration (that provides fluid communication between the container 108 and the pump 110). Activation of the pump 110 when the urine collection system is in the transfer mode T pulls the ambient air 115 through the first three-way valve 112 and the air intake 111, then forces the air through the air exhaust 113 and the second three-way valve 114 into the container 108. This flow of air creates a positive pressure in the container 108 sufficient to force the fluid in the container 108 through the second check valve 116 and the second conduit 118 and into the urine collection bag 120.

The urine collection system 100 also includes a power supply 124. The power supply 124 may be a modular power supply that is detachable from the urine collection system 100 and replaceable with a similar or different power supply. For example, a user may be plan to engage in a longer ambulatory activity, and therefore desire a longer lasting power supply. A user also may desire to switch an initial power supply to a new power supply when the initial power supply is depleted. In some embodiments, the power supply 124 is built into the pump 110. That is, the pump 110 may include a housing or compartment for the power supply 124. In some embodiments, the power supply 124 may be external to the pump 110, but may be electrically coupled to the pump 110 such that the power supply 124 may selectively power the pump 110. The power supply 124 may include one or more batteries, such as rechargeable batteries. The power supply 124 also may include a wire and plug configured to plug into a wall outlet. In some embodiments, the power supply 124 may include one or more rechargeable batteries and a wire and plug configured to charge the one or more rechargeable batteries.

The urine collection system 100 also may include a controller 122. The controller 122 may be electrically coupled to the pump 110 and the power supply 124. The controller 122 is configured to activate or deactivate the pump 110. The controller 122 also may be configured to alternate the urine collection system 100 between the collection mode C and the transfer mode T, including changing the first three-way valve 112 and the second three-way valve 122 between the respective first and second configurations. For example, the controller 122 may alternate the urine collection system 100 between the collection mode C and the transfer mode T at predetermined or preselected time intervals. In some embodiments, the urine collection system 100 includes a sensor (125) configured to detect a property related at least to a volume of the urine in the container 108, and the controller 122 may alternate the urine collection system 100 between the collection mode C and the transfer mode T responsive to a property related at least to the volume of the urine in the container 108 sensed by the sensor. For example, the sensor 125 may detect that the container is full or nearing urine capacity, and the controller 122 may alternate the urine collection system from the collection mode C to the transfer mode T. In some embodiments, the controller 122 includes one or more manual switches that allow an operator, such as the user or wearer, to activate or deactivate the pump 110 and/or change the first three-way valve 112 and the second three-way valve 122 between the respective first and second configurations.

In some embodiments, the controller 122 may include at least one computing device that may be configured to perform one or more of the acts described herein. The at least one computing device may include one or more servers, or one or more portable computing devices (e.g., smartphone, tablet, etc.). The computing device may comprise at least one processor, memory, a storage device, an input/output ("I/O") device/interface, and a communication interface. The computer device of the controller 122, then, may be configured to activate or deactivate the pump 110 and/or alternate the urine collection system 100 between the collection mode C and the transfer mode T. In some embodiments, the controller 122 may activate or deactivate the pump 110 and/or alternate the urine collection system 100 between the collection mode C and the transfer mode T in response to commands from the operator. In some embodiments, the controller 122 may automatically activate or deactivate the pump 110 and/or alternate the urine collection system 100 between the collection mode C and the transfer mode T. For example, the controller 122 may automatically activate or deactivate the pump 110 and/or alternate the urine collection system 100 between the collection mode C and the transfer mode T at predetermined or selected time intervals. The controller 122 also may activate or deactivate the pump 110 and/or alternate the urine collection system 100 between the collection mode C and the transfer mode T responsive to conditions sensed by one or more sensors in at least one of the urine collection device 102, the first conduit 104, the small reservoir 108, the second conduit 118, or the collection bag 120.

Because the urine collection system 100 may be portable for use by an ambulatory user, in embodiments, the pump 110, the power supply 124, and the fluid collection bag 120 are sized and dimensioned to be simultaneously carried and/or worn by the user when the urine collection device 102 is positioned at least proximate to the urethra of the user. In some embodiments, the pump 110 and the power supply 124 are sized and dimensioned to be detachably secured proximate to a waist of the user, and the urine collection bag 120 is sized and dimensioned to be positioned within the pocket 142 of the leg sleeve 140 worn on the leg of the user.

The collection bag 120 may be configured to be carried in a bag holder. For example, the urine collection system 100 may include a leg sleeve 140 having a pocket 142 sized to hold the collection bag 120. The pocket 142 may include may be configured to allow the collection bag 120 to expand in volume as the collection bag 120 fills with fluid. For example the pocket 142 may include an elastic material. The collection bag 120 also may be configured to be carried in a backpack 150 or waist pack. In some embodiments, one or more of the pump 110, the first three-way valve 112, the second three-way valve 114, the controller 122, or the power supply 124 also may be held or carried in the backpack 150. In some embodiments, each of the collection bag 120, the second conduit 118, the second check valve 116, the pump 110, the first three-way valve 112, the second three-way valve 114, the controller 122, or the power supply 124 are held or carried in the backpack 150. In some embodiments, each of the container 108, the pump 110, the controller 122, the power supply 124, the first three-way valve 112, and the second three-way valve 114 may be positioned in the backpack 150 or a waist pack, while the collection bag 120 is secured in the pocket 142 of the leg sleeve 140. Carrying the collection bag 120, the container 108, the pump 110, the controller 122, the power supply 124, the first three-way valve 112, and the second three-way valve 114, in one or more of the leg sleeve 140, the backpack 150, or a waist pack provides a wearer with a discreet way of collecting urine and sense of normalcy as he/she performs routine activities.

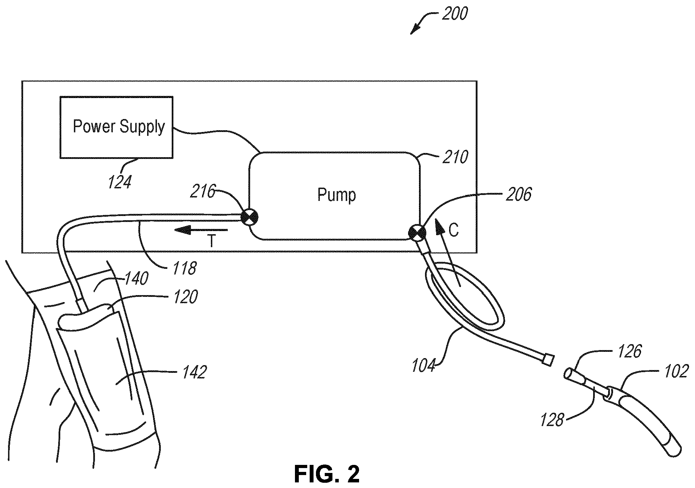

**FIG. 2** is a block diagram of a modular urine collection system 200, according to an embodiment. The urine collection system 200 includes a urine collection device 102, a first conduit 104, an additional conduit 128, a connect 126, a pump 210, a second conduit 118, a collection bag 120, and a power supply 124. Unless otherwise noted, the urine collection device 102, the first conduit 104, the pump 210, the second conduit 118, the collection bag 120, and the power supply 124 may include any of the urine collection devices 102, the first conduits 104, the additional conduits 128, the connectors, the second conduits 118, the collection bags 120, or the power supplies 124 described above in relation to the urine collection system 100. Moreover, in many embodiments, the urine collection system 200 may include the sleeve 140 and the pocket 142, as described above in relation to the urine collection system 100. In some embodiments of the urine collection system 200, one or more (*e.g.,* all) of the container 108, the first three-way valve 112, or the second three-way valve 114 of the urine collection system 100 are absent from the urine collection system 200.

The urine collection system 200 includes a modular architecture that allows one or more of the urine collection device 102, the additional conduit 128, the connector 126, the first conduit 104, the pump 210, the second conduit 118, the collection bag 120, the sleeve 140, or the pocket 142 to be replaced by a similar component or a component having features desired for specific activities. The modular architecture of the urine collection system 200 allows a patient to use the urine collection system 200 in a variety of settings, such as in bed, in a wheelchair, or ambulatory.

The pump 210 in the urine collection system 200 may vary according to different embodiments. The pump 210 may include any of the features described above in relation to the pump 110. In operation, the pump 210 pulls air or fluid through the first conduit 104 and/or the first check valve 206, then exhausts the pulled air or fluid through the second check valve 216 and/or the second conduit 118. The pump 210 may include a piezoelectric pump. In some embodiments, the pump 210 includes a disposable pump that includes the power supply 124 built into the disposable pump. When the power supply 124 in the disposable pump is depleted, a user may simply discard the disposable pump and replace the disposable pump with a new pump. The new pump replacing the disposable pump may also be a disposable pump, or may be a reusable pump. To replace the pump 210, the first conduit 104 and the second conduit 118 are disconnected from the pump 210. To replace the discarded pump 210, the first conduit 104 is connected to an intake of the new pump, and the second conduit 118 is connected to an exhaust of the new pump.

In some embodiments, the pump 210 is selectively adjustable between multiple flow rates. For example, a steady state of use by the pump 210 may include a first flow rate, while an intermittent state of use by the pump 210 may include a second flow rate that is greater than the first flow rate. The pump 210 may have a height of less than about 2.5 cm, a width of less than about 2.5 cm, and a depth of less than about 1.25 cm. The height, width, and depth of the pump 210 may vary according to different embodiments. In some embodiments, the pump 210 may include a height of less than about 1.25 cm, less than about 2.5 cm, less than about 3.75 cm, less than about 5 cm, less than about 6.25 cm, less than about 7.5 cm, about 1.25 cm to about 7.5 cm, about 1.25 cm to about 2.5 cm, about 2.5 cm to about 3.75 cm, about 3.75 cm to about 5 cm, about 5 cm to about 6.25 cm, or about 6.25 cm to about 7 cm. In some embodiments, the pump 210 may include a height of less than about 1.25 cm, less than about 2.5 cm, less than about 3.75 cm, less than about 5 cm, less than about 6.25 cm, less than about 7.5 cm, about 1.25 cm to about 7.5 cm, about 1.25 cm to about 2.5 cm, about 2.5 cm to about 3.75 cm, about 3.75 cm to about 5 cm, about 5 cm to about 6.25 cm, or about 6.25 cm to about 7 cm. In some embodiments, the pump 210 may include a depth of less than about 0.5 cm, less than about 1 cm, less than about 1.5 cm, less than about 2 cm, less than about 2.5 cm, less than about 3 cm, less than about 3.5 cm, about 0.5 cm to about 3 cm, about 0.5 cm to about 1 cm, about 1 cm to about 1.5 cm, about 1.5 cm to about 2 cm, about 2 cm to about 2.5 cm, or about 2.5 cm to about 3 cm. Embodiments may include any of the heights, widths, and/or depths provided above. In an example, the pump 210 includes a width and a height of about 2 cm and a depth of about 3 mm.

A wearer may attach the pump 210 to the collection bag 120, a bag such as a purse, a diaper, clothes (such as pants or belt), and so on. The pump 210 also may hang freely between the first conduit 104 and the second conduit 118 between the urine collection device 102 and the collection bag 120. The pump 210 also may include a first check valve 206 and a second check valve 216. The first check valve 206 is configured to provide fluid communication from the first conduit 104 into the pump 210 and prevent fluid communication from the pump 210 into the first conduit 104. The second check valve 216 is configured to provide fluid communication from the pump 210 to the second conduit 118 and prevent fluid communication from the second conduit 118 to the pump 210. In some embodiments, the pump 210 may be connected directly to a port on the collection bag 120, and the second conduit 118 is absent from the urine collection system 200. In such an embodiment, the pump 210 connected directly to the port on the collection bag 120 pulls urine from the urine collection device 102 through the first conduit 104 and directly into the fluid collection bag 120.

The collection bag 120 may include any of the collection bags 120 described in relation to the urine collection system 100. In some embodiments, the collection bag 120 includes a urinalysis test strip configured to test one or more characteristics of urine collected in the collection bag 120. The urinalysis test strip may be detachably mounted to an exterior of the collection bag 120, thereby allowing a user to detach the urinalysis test strip from the bag to test the urine at a desired time. The urinalysis test strip may be mounted to an interior of the collection bag 120, and a user may view the urinalysis test strip through a transparent wall of the collection bag, thereby allowing a user to view one or more characteristics of the urine without opening the collection bag 120. In some embodiments, the collection bag 120 includes a canister having one or more generally rigid walls.

The urine collection system 200 also includes a power supply 124. The power supply 124 may be a modular power supply that is detachable from the urine collection system 200 and replaceable with a similar or different power supply. For example, a user may be plan to engage in a longer ambulatory activity, and therefore desire a longer lasting power supply. A user also may desire to switch an initial power supply to a new power supply when the initial power supply is depleted. In some embodiments, the power supply 124 is built into the pump 210. That is, the pump 210 may include a housing or compartment for the power supply 210. In some embodiments, the power supply 124 may be external to the pump 210, but may be electrically coupled to the pump 210 such that the power supply 124 may selectively power the pump 210. The power supply 124 may include one or more batteries, such as rechargeable batteries. The power supply 124 also may include a wire and plug configured to plug into a wall outlet. In some embodiments, the power supply 124 may include one or more rechargeable batteries and a wire and plug configured to charge the one or more rechargeable batteries.

Because the urine collection system 200 may be portable for use by an ambulatory user, in embodiments, the pump 210, the power supply 124, and the fluid collection bag 120 are sized and dimensioned to be simultaneously carried and/or worn by the user when the urine collection device 102 is positioned at least proximate to the urethra of the user. In some embodiments, the pump 210 and the power supply 124 are sized and dimensioned to be detachably secured proximate to a waist of the user, and the urine collection bag 120 is sized and dimensioned to be positioned within the pocket 142 of the leg sleeve 140 worn on the leg of the user.

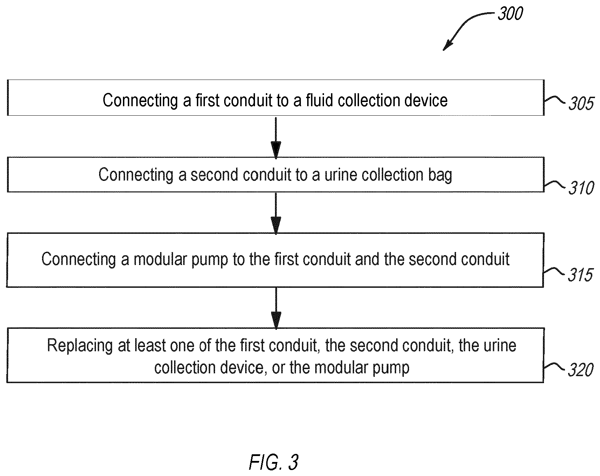

**FIG. 3** is a flow diagram of a method 300 for assembling a portable urine collection system. The method 300 includes an act 305 of connecting a first conduit to a fluid collection device to provide fluid communication between the urine collection device and the first conduit. The method 300 also includes an act 310 of connecting a second conduit to a urine collection bag to provide fluid communication between the second conduit and the urine collection bag. The method also includes an act 315 of connecting a modular pump to the first conduit and the second conduit. The modular pump is configured to pull a vacuum and draw urine from the urine collection device through the first conduit and force the urine through the second conduit into the urine collection bag. The method also includes an act 320 of replacing at least one of the first conduit, the second conduit, the urine collection device, the urine collection bag, or the modular pump.

The act 320 of replacing at least one of the first conduit, the second conduit, the urine collection device, the urine collection bag, or the modular pump may be performed after one or more portions of the portable urine collection system have been activated or used. For example, the act 320 of replacing at least one of the first conduit, the second conduit, the urine collection device, or the modular pump may be performed after the modular pump has been activated or operational for a period of time and/or at least some urine from the urine collection device has been collected in the urine collection bag,

The act 320 can include disconnecting the urine collection device from the first conduit and connecting a different urine collection device to the first conduit or a different first conduit. The act 320 can include disconnecting the second conduit from the urine collection bag and connecting a different urine collection bag to the second conduit or a different second conduit. The act 320 can include disconnecting the first conduit from the modular pump and connecting a different first conduit to the modular pump or a different modular pump. The act 320 can include disconnecting the second conduit from the modular pump and connecting a different second conduit to the modular pump or a different modular pump. The act 320 can include disconnecting the modular from the first conduit and the second conduit and connecting a different modular pump to the first conduit and the second conduit or at least one of a different first conduit or a different second conduit. The act 320 can include at least disconnecting the urine collection bag from the second conduit and connecting the different urine collection bag to the second conduit or the different second conduit.

The method 300 also may include one or more additional acts. For example, the method 300 may include disconnecting a battery from the modular pump and connecting a different battery to the modular pump or a different modular pump. The method 300 also may include testing urine collected in the urine collection bag with one or more urinalysis strips attached to the urine collection bag.

Acts 305, 310, 315, and 320 of the method 300 are for illustrative purposes. For example, the acts 305, 310, 315, and 320 of the method 300 can be performed in different orders, split into multiple acts, modified, supplemented, or combined. In an embodiment, one or more of the acts 305, 310, 315, and 320 of the method 300 can be omitted from the method 300. Any of the acts 305, 310, 315, and 320 can include using any of the portable urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting.

## Claims

1. A portable urine collection system (100) which can alternate between a collection mode (C) and a transfer mode (T) the system comprising:
a urine collection device (102) configured to be positioned at least proximate to a urethra of a user;
a container (108);
a first conduit (104) in fluid communication with the urine collection device and the container (108); **characterised in that** it further comprises:
a urine collection bag (120) having an interior region;
a second conduit (118) in fluid communication with the container (108) and the interior region of the urine collection bag; and
a modular pump (110) configured in the collection mode (C) to pull a vacuum and draw urine from the urine collection device through the first conduit and into the container (108) and in the transfer mode (T) to force the urine from the container (108) through the second conduit into the interior region of the urine collection bag (120); and
wherein:
i) the system is portable, for use in an ambulatory setting, with the pump carried in a backpack or around the waist of the user; and
ii) the system has a modular architecture with components that are interchangeable, for use of the system variously in ambulatory and non-ambulatory settings.

2. The portable urine collection system of claim 1, further comprising a battery removably connected to the modular pump.

3. The portable urine collection system of claim 1, wherein:
the container (108) is connected to the first conduit and the second conduit, the container being more rigid and having a smaller volume than the urine collection bag (120);
a first check valve (106) providing fluid communication from the first conduit into the container and preventing fluid communication from the container into the first conduit;
a second check valve (116) providing fluid communication from the container to the second conduit and preventing fluid communication from the second conduit to the container;
a first three-way valve (112) having:
a first configuration providing fluid communication between the container (108) and the modular pump (110); and
a second configuration preventing fluid communication between the container (108) and the modular pump (110), and providing fluid communication between the modular pump and ambient air; and
a second three-way valve (114) having:
a first configuration providing fluid communication between the container and the modular pump; and
a second configuration preventing fluid communication between the container and the modular pump, and providing fluid communication between the modular pump and the ambient air;
wherein:
the urine collection system can alternate between the collection mode (C) when the first three-way valve is in the first configuration and the second three-way valve is in the second configuration, activation of the modular pump pulls air through the first three-way valve into the modular pump from the container and exhausts air through the second three-way valve, thereby pulling a vacuum on the urine collection device and the first conduit to pull the urine from the urine collection device and the first conduit through the first check valve into the container; and
the transfer mode (T) when the first three-way valve is in the second configuration and the second three-way valve is in the first configuration, activation of the modular pump pulls the ambient air through the first three-way valve and forces the ambient air through the second three-way valve into the container, thereby forcing the urine in the container through the second check valve and the second conduit and into the urine collection bag.

4. The portable urine collection system of claim 3, further comprising a controller (122) operably coupled to the first three-way valve (112) and the second three-way valve (114), the controller configured to:
adjust the first three-way valve between the first configuration and the second configuration;
adjust the second three-way valve between the first configuration and the second configuration; and
activate and deactivate the pump (110).

5. The portable urine collection system of claim 4, that includes a leg sleeve (140) with a pocket (142) and the urine collection bag is sized and dimensioned to be positioned within the pocket of the leg sleeve.

6. The portable urine collection system of claim 3, further comprising a battery (124) removably connected to the modular pump and the controller, and
wherein the battery, the modular pump, the controller, the container the first three-way valve, the second three-way valve, the first check valve, and the second check valve are sized and dimensioned to simultaneously fit within a backpack (150) worn by the user.

7. The portable urine collection system of claim 6, further comprising the backpack sized and dimensioned to hold the battery, the pump, the controller, the container the first three-way valve, the second three-way valve, the first check valve, and the second check valve therein.

8. A method of assembling a portable urine collection system as claimed in any one of claims 1 to 7, the method comprising:
connecting the first conduit to the urine collection device to provide fluid communication between the urine collection device and the first conduit;
connecting the second conduit to the urine collection bag to provide fluid communication between the second conduit and the urine collection bag;
connecting the modular pump to the first conduit and the second conduit, the modular pump configured to pull a vacuum and draw urine from the urine collection device through the first conduit and force the urine through the second conduit into the urine collection bag; and
after collecting at least some urine from the urine collection device in the urine collection bag, replacing at least one of the first conduit, the second conduit, the urine collection device, the urine collection bag, and/or the modular pump with a different first conduit, a different second conduit, a different urine collection device, a different urine collection bag, and/or a different modular pump.

9. The method of claim 8, wherein replacing at least one of the first conduit, the second conduit, the urine collection device, the urine collection bag, and/or the modular pump includes at least disconnecting the urine collection device from the first conduit and connecting the different urine collection device to the first conduit and/or the different first conduit.

10. The method of either of claims 8 and 9, wherein replacing at least one of the first conduit, the second conduit, the urine collection device, the urine collection bag, and/or the modular pump includes at least disconnecting the first conduit from the modular pump and connecting the different first conduit to the modular pump and/or the different modular pump.

11. The method of any of claims 8, 9 and 10, wherein replacing at least one of the first conduit, the second conduit, the urine collection device, the urine collection bag, or the modular pump includes at least disconnecting the second conduit from the modular pump and connecting the different second conduit to the modular pump and/or the different modular pump.

12. The method of any of claims 8 to 11, wherein replacing at least one of the first conduit, the second conduit, the urine collection device, the urine collection bag, and/or the modular pump includes at least disconnecting the modular pump from the first conduit and the second conduit and connecting the different modular pump to the first conduit and the second conduit.

13. The method of any of claims 8 to 12, further comprising inserting the modular pump and/or the different modular pump into a backpack configured to be worn by the user.

14. The method of any of claims 8 to 12, further comprising securing the modular pump proximate to the waist of the user.

15. The portable urine collection system as claimed in any one of claims 1 to 7 including a power supply which includes one or more rechargeable batteries and a wire and plug configured to charge the batteries.

## Patentansprüche

1. Tragbares Urinsammelsystem (100), das zwischen einem Sammelmodus (C) und einem Transfermodus (T) alternieren kann, wobei das System Folgendes umfasst:
eine Urinsammelvorrichtung (102), die so ausgebildet ist, dass sie zumindest in der Nähe einer Harnröhre eines Benutzers positioniert wird;
einen Behälter (108);
eine erste Leitung (104) in Fluidverbindung mit der Urinsammelvorrichtung und dem Behälter (108); **dadurch gekennzeichnet, dass** es weiter Folgendes umfasst:
einen Urinsammelbeutel (120), der einen Innenbereich aufweist;
eine zweite Leitung (118) in Fluidverbindung mit dem Behälter (108) und dem Innenbereich des Urinsammelbeutels; und
eine modulare Pumpe (110), die im Sammelmodus (C) so ausgebildet ist, dass sie ein Vakuum anlegt und Urin aus der Urinsammelvorrichtung durch die erste Leitung und in den Behälter (108) zieht und im Transfermodus (T) so ausgebildet ist, dass sie den Urin aus dem Behälter (108) durch die zweite Leitung in den Innenraum des Urinsammelbeutels (120) drängt; und
wobei:
i) das System tragbar ist, zur Verwendung in einer ambulanten Umgebung, wobei die Pumpe in einem Rucksack oder um die Taille des Benutzers getragen wird; und
ii) das System eine modulare Architektur mit Komponenten aufweist, die austauschbar sind, zur unterschiedlichen Verwendung des Systems in ambulanten und nicht-ambulanten Umgebungen.

2. Tragbares Urinsammelsystem nach Anspruch 1, weiter umfassend eine Batterie, die abnehmbar mit der modularen Pumpe verbunden ist.

3. Tragbares Urinsammelsystem nach Anspruch 1, wobei:
der Behälter (108) mit der ersten Leitung und der zweiten Leitung verbunden ist, wobei der Behälter steifer ist und ein kleineres Volumen als der Urinsammelbeutel (120) aufweist;
wobei ein erstes Rückschlagventil (106) eine Fluidverbindung von der ersten Leitung in den Behälter bereitstellt und eine Fluidverbindung von dem Behälter in die erste Leitung verhindert;
wobei ein zweites Rückschlagventil (116) eine Fluidverbindung von dem Behälter zu der zweiten Leitung bereitstellt und eine Fluidverbindung von der zweiten Leitung zu dem Behälter verhindert;
wobei ein erstes Dreiwegeventil (112) Folgendes aufweist:
eine erste Konfiguration, die eine Fluidverbindung zwischen dem Behälter (108) und der modularen Pumpe (110) bereitstellt; und
eine zweite Konfiguration, die eine Fluidverbindung zwischen dem Behälter (108) und der modularen Pumpe (110) verhindert und eine Fluidverbindung zwischen der modularen Pumpe und der Umgebungsluft bereitstellt; und
ein zweites Dreiwegeventil (114), aufweisend:
eine erste Konfiguration, die eine Fluidverbindung zwischen dem Behälter und der modularen Pumpe bereitstellt; und
eine zweite Konfiguration, die eine Fluidverbindung zwischen dem Behälter und der modularen Pumpe verhindert und eine Fluidverbindung zwischen der modularen Pumpe und der Umgebungsluft bereitstellt;
wobei:
das Urinsammelsystem alternieren kann zwischen dem Sammelmodus (C), wenn sich das erste Dreiwegeventil in der ersten Konfiguration und das zweite Dreiwegeventil in der zweiten Konfiguration befindet, wobei Aktivierung der modularen Pumpe Luft durch das erste Dreiwegeventil aus dem Behälter in die modulare Pumpe zieht und Luft durch das zweite Dreiwegeventil ablässt, wodurch ein Vakuum an der Urinsammelvorrichtung und der ersten Leitung angelegt wird, um den Urin aus der Urinsammelvorrichtung und der ersten Leitung durch das erste Rückschlagventil in den Behälter zu ziehen; und
dem Transfermodus (T), wenn das erste Dreiwegeventil in der zweiten Konfiguration ist und das zweite Dreiwegeventil in der ersten Konfiguration ist, wobei Aktivierung der modularen Pumpe, die Umgebungsluft durch das erste Dreiwegeventil zieht und die Umgebungsluft durch das zweite Dreiwegeventil in den Behälter drückt, wodurch der Urin im Behälter durch das zweite Rückschlagventil und die zweite Leitung und in den Urinsammelbeutel gedrückt wird.

4. Tragbares Urinsammelsystem nach Anspruch 3, weiter umfassend eine Steuereinheit (122), die betriebsfähig mit dem ersten Dreiwegeventil (112) und dem zweiten Dreiwegeventil (114) gekoppelt ist, wobei die Steuereinheit ausgebildet ist zu Folgendem:
Einstellen des ersten Dreiwegeventils zwischen der ersten Konfiguration und der zweiten Konfiguration;
Einstellen des zweiten Dreiwegeventils zwischen der ersten Konfiguration und der zweiten Konfiguration; und
Aktivieren und Deaktivieren der Pumpe (110).

5. Tragbares Urinsammelsystem nach Anspruch 4, das eine Beinmanschette (140) mit einer Tasche (142) einschließt, und wobei der Urinsammelbeutel so bemessen und dimensioniert ist, dass er in der Tasche der Beinmanschette positioniert wird.

6. Tragbares Urinsammelsystem nach Anspruch 3, weiter umfassend eine Batterie (124), die abnehmbar mit der modularen Pumpe und der Steuereinheit verbunden ist, und
wobei die Batterie, die modulare Pumpe, die Steuereinheit, der Behälter, das erste Dreiwegeventil, das zweite Dreiwegeventil, das erste Rückschlagventil und das zweite Rückschlagventil so bemessen und dimensioniert sind, dass sie gleichzeitig in einen von dem Benutzer getragenen Rucksack (150) passen.

7. Tragbares Urinsammelsystem nach Anspruch 6, weiter umfassend den Rucksack, der so bemessen und dimensioniert ist, dass er die Batterie, die Pumpe, die Steuereinheit, den Behälter, das erste Dreiwegeventil, das zweite Dreiwegeventil, das erste Rückschlagventil und das zweite Rückschlagventil aufnimmt.

8. Verfahren zum Zusammenbauen eines tragbaren Urinsammelsystems nach einem der Ansprüche 1 bis 7, wobei das Verfahren Folgendes umfasst:
Verbinden der ersten Leitung mit der Urinsammelvorrichtung, um eine Fluidverbindung zwischen der Urinsammelvorrichtung und der ersten Leitung bereitzustellen;
Verbinden der zweiten Leitung mit dem Urinsammelbeutel, um eine Fluidverbindung zwischen der zweiten Leitung und dem Urinsammelbeutel bereitzustellen;
Verbinden der modularen Pumpe mit der ersten Leitung und der zweiten Leitung, wobei die modulare Pumpe so ausgebildet ist, dass sie ein Vakuum anlegt und Urin aus der Urinsammelvorrichtung durch die erste Leitung zieht und den Urin durch die zweite Leitung in den Urinsammelbeutel drängt; und
nach dem Sammeln von mindestens etwas Urin aus der Urinsammelvorrichtung in dem Urinsammelbeutel, Ersetzen mindestens einer von der ersten Leitung, der zweiten Leitung, der Urinsammelvorrichtung, des Urinsammelbeutels und/oder der modularen Pumpe durch eine andere erste Leitung, eine andere zweite Leitung, eine andere Urinsammelvorrichtung, einen anderen Urinsammelbeutel und/oder eine andere modulare Pumpe.

9. Verfahren nach Anspruch 8, wobei das Ersetzen von mindestens einer der ersten Leitung, der zweiten Leitung, der Urinsammelvorrichtung, des Urinsammelbeutels und/oder der modularen Pumpe mindestens das Trennen der Urinsammelvorrichtung von der ersten Leitung und das Verbinden der anderen Urinsammelvorrichtung mit der ersten Leitung und/oder der anderen ersten Leitung einschließt.

10. Verfahren nach einem der Ansprüche 8 und 9, wobei das Ersetzen von mindestens einer der ersten Leitung, der zweiten Leitung, der Urinsammelvorrichtung, des Urinsammelbeutels und/oder der modularen Pumpe mindestens das Trennen der ersten Leitung von der modularen Pumpe und das Verbinden der anderen ersten Leitung mit der modularen Pumpe und/oder der anderen modularen Pumpe einschließt.

11. Verfahren nach einem der Ansprüche 8, 9 und 10, wobei das Ersetzen von mindestens einer der ersten Leitung, der zweiten Leitung, der Urinsammelvorrichtung, des Urinsammelbeutels oder der modularen Pumpe mindestens das Trennen der zweiten Leitung von der modularen Pumpe und das Verbinden der anderen zweiten Leitung mit der modularen Pumpe und/oder der anderen modularen Pumpe einschließt.

12. Verfahren nach einem der Ansprüche 8 bis 11, wobei das Ersetzen von mindestens einer der ersten Leitung, der zweiten Leitung, der Urinsammelvorrichtung, des Urinsammelbeutels und/oder der modularen Pumpe mindestens das Trennen der modularen Pumpe von der ersten Leitung und der zweiten Leitung und das Verbinden der anderen modularen Pumpe mit der ersten Leitung und der zweiten Leitung einschließt.

13. Verfahren nach einem der Ansprüche 8 bis 12, weiter umfassend das Einsetzen der modularen Pumpe und/oder der anderen modularen Pumpe in einen Rucksack, der so ausgebildet ist, dass er von dem Benutzer getragen wird.

14. Verfahren nach einem der Ansprüche 8 bis 12, weiter umfassend das Befestigen der modularen Pumpe in der Nähe der Taille des Benutzers.

15. Tragbares Urinsammelsystem nach einem der Ansprüche 1 bis 7, das eine Stromversorgung einschließt, die eine oder mehrere wiederaufladbare Batterien und eine Leitung und einen Stecker einschließt, die dazu ausgebildet sind, die Batterien zu laden.

## Revendications

1. Système portable (100) de collecte d'urine, qui est apte à alterner entre un mode de collecte (C) et un mode de transfert (T), le système comprenant :
un dispositif (102) de collecte d'urine, conçu pour être placé au moins à proximité de l'urètre d'un utilisateur ;
un récipient (108) ;
un premier conduit (104), en communication fluidique avec le dispositif de collecte d'urine et le récipient (108) ; **caractérisé en ce qu'**il comprend en outre :
une poche (120) de collecte d'urine, présentant une partie intérieure ;
un deuxième conduit (118), en communication fluidique avec le récipient (108) et la partie intérieure de la poche de collecte d'urine ; et
une pompe modulaire (110), configurée, dans le mode de collecte (C), de manière à créer une dépression et aspirer l'urine depuis le dispositif de collecte d'urine à travers le premier conduit et dans le récipient (108) et, dans le mode de transfert (T), de manière à forcer l'urine depuis le récipient (108) à travers le deuxième conduit dans la partie intérieure de la poche (120) de collecte d'urine ; et
dans lequel :
i) le système est portable, pour une utilisation dans un cadre ambulatoire, avec la pompe portée dans un sac à dos ou autour de la taille de l'utilisateur ; et
ii) le système présente une architecture modulaire avec des composants qui sont interchangeables, pour une utilisation du système de manière variée dans des cadres ambulatoires et non ambulatoires.

2. Système de collecte d'urine portable selon la revendication 1, comprenant en outre une batterie reliée de manière amovible à la pompe modulaire.

3. Système de collecte d'urine portable selon la revendication 1, dans lequel :
le récipient (108) est relié au premier conduit et au deuxième conduit, le récipient étant plus rigide et présentant un volume plus petit que la poche (120) de collecte d'urine ;
un premier clapet anti-retour (106) assure une communication de fluide entre le premier conduit et le récipient et empêche la communication de fluide entre le récipient et le premier conduit ;
un deuxième clapet anti-retour (116) assure une communication fluidique depuis le récipient vers le deuxième conduit et empêche toute communication fluidique depuis le deuxième conduit vers le récipient ;
une première vanne à trois voies (112) présentant :
une première configuration, fournissant une communication fluidique entre le récipient (108) et la pompe modulaire (110) ; et
une deuxième configuration, empêchant une communication fluidique entre le récipient (108) et la pompe modulaire (110), et fournissant une communication fluidique entre la pompe modulaire et l'air ambiant ; et
une deuxième vanne à trois voies (114), présentant :
une première configuration, assurant une communication fluidique entre le récipient et la pompe modulaire ; et
une deuxième configuration, empêchant toute communication fluidique entre le récipient et la pompe modulaire, et établissant une communication fluidique entre la pompe modulaire et l'air ambiant ;
dans lequel :
le système de collecte d'urine est apte à alterner entre le mode de collecte (C) lorsque la première vanne à trois voies est dans la première configuration et la deuxième vanne à trois voies est dans la deuxième configuration, l'activation de la pompe modulaire tirant de l'air à travers la première vanne à trois voies dans la pompe modulaire depuis le récipient et évacuant de l'air à travers la deuxième vanne à trois voies, créant ainsi une dépression au niveau du dispositif de collecte d'urine et du premier conduit afin d'aspirer l'urine du dispositif de collecte d'urine et du premier conduit à travers le premier clapet anti-retour vers le récipient ; et
le mode de transfert (T) lorsque la première vanne à trois voies est dans la deuxième configuration et la deuxième vanne à trois voies est dans la première configuration, l'activation de la pompe modulaire tirant l'air ambiant à travers la première vanne à trois voies et forçant l'air ambiant à travers la deuxième vanne à trois voies dans le récipient, forçant ainsi l'urine dans le récipient à travers le deuxième clapet anti-retour et le deuxième conduit et dans la poche de collecte d'urine.

4. Système de collecte d'urine portable selon la revendication 3, comprenant en outre un dispositif de commande (122) relié fonctionnellement à la première vanne à trois voies (112) et à la deuxième vanne à trois voies (114),
le dispositif de commande est configuré pour :
régler la première vanne à trois voies entre la première configuration et la deuxième configuration ;
régler la deuxième vanne à trois voies entre la première configuration et la deuxième configuration ; et
activer et désactiver la pompe (110).

5. Système de collecte d'urine portable selon la revendication 4, qui inclut un manchon de jambe (140) avec une poche (142), et la poche de collecte d'urine est dimensionnée et agencée de manière à être positionnée à l'intérieur de la poche du manchon de jambe.

6. Système de collecte d'urine portable selon la revendication 3, comprenant en outre une batterie (124) reliée de manière amovible à la pompe modulaire et au dispositif de commande, et
dans lequel la batterie, la pompe modulaire, le dispositif de commande, le récipient, la première vanne à trois voies, la deuxième vanne à trois voies, le premier clapet anti-retour et le deuxième clapet anti-retour sont dimensionnés de manière à pouvoir tenir simultanément dans un sac à dos (150) porté par l'utilisateur.

7. Système de collecte d'urine portable selon la revendication 6, comprenant en outre un sac à dos dont la taille et les dimensions sont adaptées pour contenir la batterie, la pompe, le dispositif de commande, le récipient, la première vanne à trois voies, la deuxième vanne à trois voies, le premier clapet anti-retour et le deuxième clapet anti-retour.

8. Procédé d'assemblage d'un système de collecte d'urine portable selon l'une quelconque des revendications 1 à 7, le procédé comprenant :
le fait de relier le premier conduit au dispositif de collecte d'urine pour fournir une communication fluidique entre le dispositif de collecte d'urine et le premier conduit ;
le fait de relier le deuxième conduit à la poche de collecte d'urine pour fournir une communication fluidique entre le deuxième conduit et la poche de collecte d'urine ;
le fait de relier la pompe modulaire au premier conduit et au deuxième conduit, la pompe modulaire étant configurée pour créer une dépression et aspirer l'urine depuis le dispositif de collecte d'urine à travers le premier conduit et forcer l'urine à travers le deuxième conduit dans la poche de collecte d'urine ; et
après avoir recueilli au moins une partie de l'urine provenant du dispositif de collecte d'urine dans la poche de collecte d'urine, le fait de remplacer au moins un parmi le premier conduit, le deuxième conduit, le dispositif de collecte d'urine, la poche de collecte d'urine et/ou la pompe modulaire par un premier conduit différent, un deuxième conduit différent, un dispositif de collecte d'urine différent, une poche de collecte d'urine différente et/ou une pompe modulaire différente.

9. Procédé selon la revendication 8, dans lequel le remplacement d'au moins un parmi le premier conduit, le deuxième conduit, le dispositif de collecte d'urine, la poche de collecte d'urine, et/ou la pompe modulaire inclut au moins le fait de déconnecter le dispositif de collecte d'urine du premier conduit et de relier le dispositif de collecte d'urine différent au premier conduit et/ou au premier conduit différent.

10. Procédé selon l'une ou l'autre des revendications 8 et 9, dans lequel le remplacement d'au moins un parmi le premier conduit, le deuxième conduit, le dispositif de collecte d'urine, la poche de collecte d'urine, et/ou la pompe modulaire inclut au moins le fait de déconnecter le premier conduit de la pompe modulaire et de relier le premier conduit différent à la pompe modulaire et/ou à la pompe modulaire différente.

11. Procédé selon l'une quelconque des revendications 8, 9 et 10, dans lequel le remplacement d'au moins un parmi le premier conduit, le deuxième conduit, le dispositif de collecte d'urine, la poche de collecte d'urine, ou la pompe modulaire inclut au moins le fait de déconnecter le deuxième conduit de la pompe modulaire et de relier le deuxième conduit différent à la pompe modulaire et/ou à la pompe modulaire différente.

12. Procédé selon l'une quelconque des revendications 8 à 11, dans lequel le remplacement d'au moins un parmi le premier conduit, le deuxième conduit, le dispositif de collecte d'urine, la poche de collecte d'urine, et/ou la pompe modulaire inclut au moins le fait de déconnecter la pompe modulaire du premier conduit et du deuxième conduit et de relier la pompe modulaire différente au premier conduit et au deuxième conduit.

13. Procédé selon l'une quelconque des revendications 8 à 12, comprenant en outre le fait d'insérer la pompe modulaire et/ou la pompe modulaire différente dans un sac à dos conçu pour être porté par l'utilisateur.

14. Procédé selon l'une quelconque des revendications 8 à 12, comprenant en outre le fait de fixer la pompe modulaire à proximité de la taille de l'utilisateur.

15. Système de collecte d'urine portable selon l'une quelconque des revendications 1 à 7 incluant une alimentation électrique qui inclut une ou plusieurs batteries rechargeables et un fil et une fiche configurés pour charger les batteries.
